# EUROPEAN PATENT SPECIFICATION

(11) **EP 0 432 218 B1**
(45) Date of publication and mention of the grant of the patent: **28.06.1995**
(21) Application number: 89910554.8
(22) Date of filing: 30.08.1989
(51) Int. Cl.: G01N 33/53, G01N 33/559, G01N 33/543, G01N 33/549, G01N 33/68, G01N 33/50, G01N 33/569

(54) **METHOD FOR DETECTING CELLULAR PATHOLOGY**
VERFAHREN ZUM NACHWEIS DER ZELLULARER PATHOLOGIE
PROCEDE DE DETECTION DE PATHOLOGIE CELLULAIRE

(30) Priority: 02.09.1988 US 240154
(43) Date of publication of application: 19.06.1991
(73) Proprietor: THE REGENTS OF THE UNIVERSITY OF CALIFORNIA, Oakland, California 94612-3550 (US)
(72) Inventor: LYNCH, Gary, S., Irvine, CA 92715 (US); SEUBERT, Peter, A., San Francisco, CA 94080 (US)
(74) Representative: UEXKÜLL & STOLBERG
(86) International application number: US8903748
(87) International publication number: WO9002949

(56) References cited:
- PROC. NATL. ACAD. SCI. USA, vol. 81, February 1984, pages 776-780, Washington, DC, US; S.T. APPLEYARD et al.: "Monoclonal antibodies detect a spectrin-like protein in normal and dystrophic human skeletal muscle"
- BIOCHEMICAL PHARMACOLOGY, vol. 34, no. 24, 1985, pages 4283-4289, New York, US; A. ARDUINI et al.: "Spectrin degradation in intact red blood cells by phenylhydrazine"
- BRAIN RESEARCH, vol. 459, no. 2, 6th September 1988, pages 226-232, Elsevier Science Publishers B.V. (Biomedical Division), Amsterdam, NL; P. SEUBERT et al.: "Lesions of entorhinal cortex produce a calpain-mediated degradation of brain spectrin in dentate gyrus. I. Biochemical studies"
- BRAIN RESEARCH, vol. 460, no. 1, 13th September 1988, pages 189-194, Elsevier Science Publishers B.V. (Biomedical Division), Amsterdam, NL; P. SEUBERT et al.: "Stimulation of NMDA receptors induces proteolysis of spectrin in hippocampus"
- PROC. NATL. ACAD. SCI. USA, vol. 81, 1984, pages 3572-3576, see p. 3573 (Methods) and p. 3574, column 1, lines 8-26; R. SIMAN et al.: "Brain fodrin:Substrate for calpain I, an endogenous calcium-activated protease".
- BLOOD, vol. 69, No. 2, 1987, pages 537-547, see the Introduction; J.E.B. FOX et al.: "Spectrin is Associated with Membrane-Bound Action Filaments inPlaletets".
- PROC. NATL. ACAD. SCI, USA, Vol. 79, 1982, pages 4002-4005, see the Abstract and Introduction; J.R. GLENNEY et al.: "Erythroid spectrin, brain fodrin and intestinal brush border-proteins (TW-260/240) are related molecules containing a common calmodulin-binding subunit bound to a variant cell type-specific subunit".
- BIOCHIM, BIOPHYSICA ACTA, Vol. 830, 1985, pages 147-158, See the Abstract and Introduction; A.S. HARRIS et al.: "Mechanism of cytgoskeletal regulation (I). Functional differences correlate with antigenic dissimilarity in human brain and erythrocyte spectrin".
- J. BIOL. CHEM, Vol. 258, No. 12, 1983, pages 7757-7767; J. DAVIS et al.: "Brain Spectrin".

## Description

### BACKGROUND OF THE INVENTION

This invention relates to an in vitro method for the detection of cellular pathology, and more specifically to an assay for monitoring cytoskeleton breakdown products to determine cell necrosis.

This invention was made with government support under AFSOR Contract No. 86-0099 (P.I.: Lynch), NIH Grant No. NS-18427 and NIA Grant No. AG00538. The government has certain rights in this invention.

The structural integrity of cells is maintained in part by the cytoskeleton, a mesh-like structure composed primarily of proteins, which lies adjacent to the inner cell surface. The cytoskeleton of many cell types (a partial list includes neurons, lymphocytes, kidney, liver, cardiac and smooth muscle, and blood platelets) contain large amounts of a protein either identical to or closely related to brain spectrin (also known as fodrin). Calpain is an enzyme which in the presence of elevated calcium levels degrades spectrin, thereby leading to the disruption of the cytoskeleton.

A variety of cellular insults (e.g., toxins, anoxia, etc.) and disease states (e.g., Alzheimer's, Parkinson's, muscular dystrophy) cause the degeneration and death of cells. Often, however, it is not possible to determine that injury has occurred until degenerative effects are irreversible. There thus exists a need for a reliable method to detect degenerative events as soon as possible after their onset before pathological symptoms appear. Preferably such method should have high sensitivity, wide ranging applicability and ease of administration. The present invention satisfies this need and provides related advantages.

### SUMMARY OF THE INVENTION

The invention provides a method for the detection of cellular pathology by means of an immunoassay to determine the presence of stable breakdown products, termed BDP's or BDP1 and BDP2, of the cytoskeleton component spectrin. In one aspect of the invention, the components from a sample of spectrin-containing cells are physically separated, as by exposure to an electric field, in such a way that BDP and spectrin are separated. Antibodies reactive with BDP are then contacted with the separated sample, and their binding to that portion of the sample containing any BDP determined.

### BRIEF DESCRIPTION OF THE DRAWINGS

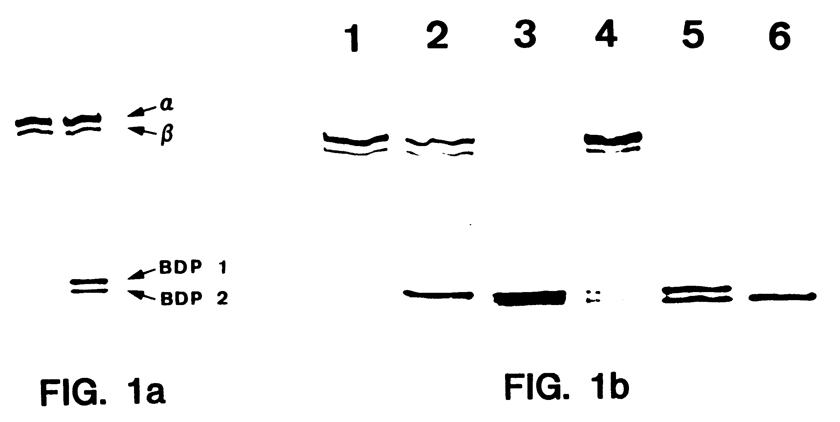

Figure 1. A. Spectrin immunoreactivity in blotted samples of the contralateral (left lane) and ipsilateral (right lane) dentate gyrus two days after a unilateral lesion of the entorhinal cortex. Arrows indicate the alpha and beta spectrin subunits with apparent Mᵣs of about 240 and 230 KDa respectively and two additional immunoreactive peptides (BDP1 and BDP2) with apparent Mᵣs of about 155 and 150 KDa respectively.

B. Purified brain spectrin incubated under the following conditions. Lane 1: no additions; Lane 2: lmM CaCl₂, 1.8 »g/ml calpain I, 10 minutes; Lane 3: 1mM CaCl₂, 3 »g/ml calpain I, 30 minutes; Lane 4: 10 »g of dentate gyrus protein homogenate two days post-lesion. Lane 5: lmM CaCl₂, 13 »g/ml calpain, 1.25 »g/ml colmodulin, 3 minutes; Lane 6: 1mM CaCl₂, 13 »g/ml calpain, 1.25 »g/ml colmodulin, 30 minutes.

Figure 2. BDP1 (filled circles) and BDP2 (open circles) levels are expressed as a percentage of the total spectrin immunoreactivity, as determined by scanning reflective densitometry.

Figure 3. Levels of BDP's in regions of the brain of control and Brindled mice, showing the effect of treatment with copper.

Figure 4. Levels of BDP in the dentate gyrus and the CAl region of the hippocampus of rats receiving trimethyltin.

Figure 5. Levels of BDP in the dentate gyrus and the CA1 region of the hippocampus from a gerbil following ischemia.

### DETAILED DESCRIPTION OF THE PREFERRED EMBODIMENT

The present invention relates to a sensitive and efficient method for the early detection of cellular death and degradation. The method involves detecting cellular death or degradation in a subject by analyzing a biological sample for the presence of spectrin breakdown products and comparing the quantity of spectrin breakdown products to the quantity of spectrin breakdown products in a normal subject, such that an increased level of spectrin breakdown products indicates cellular death or degradation in the subject. The method can be carried out, for example, by exposing the sample to an electrical gradient so as to separate the components in such a way that spectrin breakdown products are separated from spectrin, followed by staining the separated products so as to visualize the products and determine the presence of stain binding. Alternatively, or in addition thereto, the method involves an immuno assay using antibodies which recognize stable breakdown products of spectrin".

Spectrin binds F-actin, and together they are generally associated with the inner face of the cell membrane, where they form a filamentous meshwork. Brain and many other tissues have been known for some time to express calcium-stimulated proteolytic activity. Studies of degeneration in peripheral nerves have indicated that a calcium-dependent protease, termed calpain, is critically involved in the degradation of neurofilament proteins following denervation or injury. Two forms of this protease have been identified in the brain and other tissues which are differentiated by their threshold for activation by calcium: calpain I requires micromolar calcium while calpain II is activated by concentrations of calcium between 0.1 and 0.5 mM. The two forms are differentially distributed in the brain. While calpain II is mainly localized in the cytoplasmic fraction of brain cells, the highest activity of calpain I is found in small processes.

Brain spectrin can be purified to greater than 90% purity by the method of Davis and Bennett, J. Biol. Chem. 258:7757-7766 (1983), which is incorporated herein by reference. Calpain can be purified to a similar level of purity from rat erythrocyte cytosol according to the method of Seubert et al., Synapse 1:20-24 (1987), which is incorporated herein by reference.

In order to identify spectrin breakdown products, 75 »g/ml of spectrin was incubated at 30°C with 100 uM CaCl₂, 3 »g/ml calpain I, 20 mM Tris-Cl, 5mM mercaptoethanol, and 150 mM NaCl at pH 7.5. Aliquots were withdrawn at various times and added to one-third volume of 3X's SDS-PAGE buffer (150mM Tris-Po₄, 6%, SDS, 30% glycerol, 3.75mM EDTA, 3% mercaptoethanol, pH 6.8). The samples were heated in 90°C water bath for 3-10 minutes, and subjected to SDS-PAGE on 3 to 10% gradient gels. The gels were stained with Coomassie blue and restained with 7% acetic acid. See Fig. 1, Seubert et al. Synapse 1:20-24 (1987), which is incorporated herein by reference. Peptide bands of approximately 150,000 Daltons were found to increase as spectrin disappeared. The peptides of these bands were termed BDP's. The apparent stability of the BDP's towards further degradation suggested that antibodies directed against spectrin may recognize the BDP's in biological samples, such as tissues, fluids, etc.

Antibodies to brain spectrin, which are also reactive with BDP1 and BDP2, were raised in rabbits using well known procedures (See, for example, Hurn, B.A.L. and Chantler, S.M. Meth. Enz. 70:104-135 (1980) which is incorporated herein by reference). The anti-brain spectrin antibodies were purified from serum by brain spectrin-sepharose affinity chromatography using methods well-known in the art.

Purified brain spectrin was incubated as described for Figure 1B. After SDS-PAGE, the proteins were electrophoretically transferred to a nitro cellulose membrane using an Immuno-Blot Assay Kit (Bio-Rad, Richmond, CA) according to the manufacturer's recommendations for the transfer of high molecular weight proteins. As shown in Figure 1B, in the presence of Calpain the degradation of spectrin produces BDP's, primarily BDP1 and BDP2.

The following examples are intended to illustrate but not limit the invention. While they are typical of those that might be used, other alternative procedures known to those skilled in the art may be alternatively employed.

### EXAMPLE I

### BDP1 and BDP2 RESULTING FROM BRAIN LESIONS

Injury in the mammalian Central Nervous System (CNS) results in both the degeneration of damaged neurons and growth responses of undamaged neuronal elements. A well-documented paradigm for investigating the mechanisms underlying these processes involves lesioning the entorhinal cortex, resulting in the production of a well-defined dendritic zone in the dentate gyrus deprived of the majority of its axonal inputs. The anatomical consequences of denervation include dendritic atrophy, glial hypertrophy and atrophy, and a growth response in undamaged axons. In addition, aberrations in cytoplasmic calcium levels occur; see, for example, Baudry, et al., J. Neurosci 3:252-259 (1983), which is incorporated herein by reference.

Stereotaxically placed unilateral electrolytic lesions of the entorhinal cortex were made in Sprague-Dawley rats. Animals were sacrificed after postoperative survival times of 0.2, 0.5, 1, 2, 4, 7, 14 and 27 days.

Immediately after sacrificing the animals by decapitation, brains were rapidly dissected in ice-cold homogenization buffer consisting of 0.32 M sucrose, 10mM Tris, 2mM EDTA, lmM ethylene glycol-bis B-amino-ethylether (EGTA) N,N,N′,N′-tetraacetic acid, 100 »M leupetin, 1 »g/ml N-tosyl-L-phenylalanine chloromethyl ketone (TPCK), pH 7.4 Each hippocampus was dissected free and cuts were made with a scalpel blade to isolate the dentate gyrus. With the hippocampus resting on the alvear surface, one cut was made longitudinally along the hippocampal fissure to separate the subiculum and another longitudinal cut removed most of the CA3 field. A third cut was then made in CA1, parallel to the fissure, to remove the majority of CA1. The remaining tissue (10-20 mg) served as the dentate gyrus sample.

The dissected dentate gyrus was homogenized in 500 »l of dissection buffer. An aliquot was added to one-third volume of 3X's SDS-PAGE sample buffer (consisting of 150mM Tris-Po₄, 5%, SDS, 30% glycerol, 3.75mM EDTA, 3% mercaptoethanol, pH 6.8) and placed in a boiling water bath for three minutes. The protein concentration of the homogenate was determined by the method of Bradford, Anal. Biochem. 72:248-254 (1976), which is incorporated herein by reference, and the concentration of proteins in sample buffer adjusted to 0.33 mg/ml. Ten »g of each sample was subjected to SDS polyacrylamide gel electrophoresis on 3-10% gradient gels until a bromophenol marker dye reached the front of the gel. The proteins were then transferred to nitrocellulose membrane using a transblot apparatus (BioRad, Richmond, CA) according to the manufacturer's instructions for high molecular weight proteins.

Antibodies were produced by the following method: For each rabbit approximately 200 »g of purified brain spectrin was excised from SDS-polyacrylamide gels (after electrophoretic separation) and emulsified with Freund's complete adjuvant. Multiple subdermal injections were made and the procedure repeated again after 2 to 4 weeks, using Freund's Incomplete Adjuvant. After an additional two weeks subcutaneous injections of an emulsion containing approximately 100 »g of spectrin were made. This procedure was repeated approximately one month later. Ten days following this series of injections, approximately 20 ml of blood was drawn from the rabbit and the serum collected after allowing the blood to clot overnight at 4°C.

Antibodies to brain spectrin were isolated from the serum by adsorption to brain spectrin which has been coupled to 6-amino hexanoic acid activated sepharose 4B (Sigma Chemical Co., St. Louis, MO). The specifically bound antibodies were then eluted in 0.2 M glycine, pH 2.8. The affinity purified antibodies were then equilibrated to pH 7.4 and frozen until use. The procedures for blocking, primary and secondary antibody incubations and color development were as recommended by BioRad (Bio-Rad Immuno-Blot™ Assay Kit: Instruction Manual) using anti-rabbit IgG (Bio-Rad, Richmond, CA) conjugated to alkaline phosphatase with the 5-bromo-4-chloro-3-indolyl phosphate/nitro blue tetrazolium substrate system of detection, according to the manufacturer's recommendations. Affinity purified anti-spectrin antibody was diluted 1/750 (in a volume of 50 ml) and incubated overnight with the blot during the primary antibody step. Quantitation of the immunoreactive species was made using a soft laser scanning densitometer (Model #SLR504-XL, BioMed Instruments, Fullerton, CA). An integrator 4270 Varion, Sunnyvale, CA) summed the amount of reaction product in each band and expressed them as a % of the total in that sample.

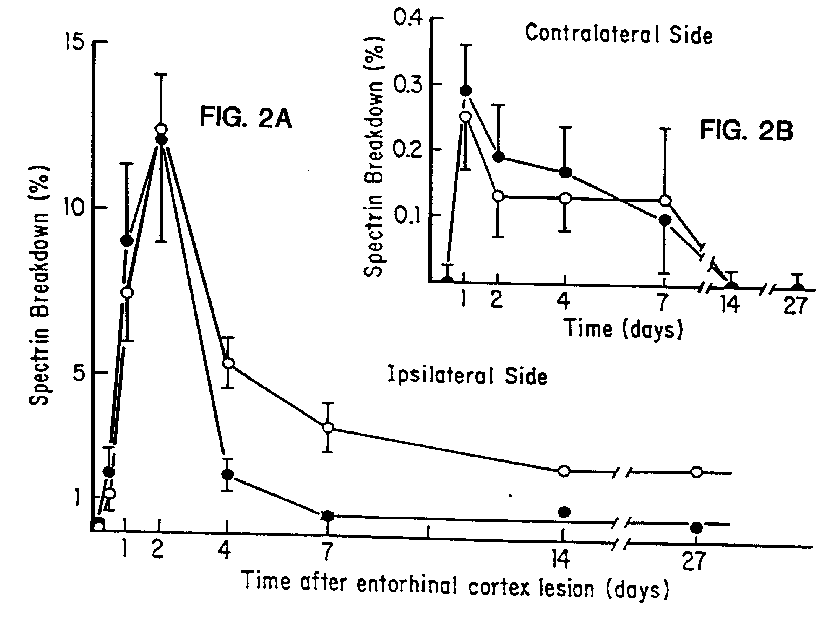

The anti-spectrin reactive species present in the dentate gyrus two days after a unilateral entorhinal lesion ar shown in Figure 1A. The homogenates of the ipsilateral dentate gyrus exhibited a marked increase in the amount of two peptides, termed BDP1 and BDP2, with apparent MᵣS of about 155,000 and 150,000 Daltons respectively. The time course of the changes in BDP1 and BDP2 in the dentate gyrus following a unilateral entorhinal cortex lesion is shown in Figure 2. BDP's are usually below the limit of detection in samples from unoperated animals. A significant elevation of BDP's in the ipsilateral sample is evident as early as four hours post-lesion. The increase is maximal two days after the lesion, where the BDP's represent 25% of the total immunoreactivity. Two and even four weeks after the lesion, the amounts of BDP's were still significantly increased; the contralateral dentate gyri at two and four weeks showed no detectable BDP's and average BDP2 levels were less than 0.1%. Small increases in the amounts of BDP's were observed in the contralateral region during the first week following the lesion as compared to non-operated animals. These results indicate that removal of the main input to the dentate gyrus is followed by a rapid and longlasting increased degradation of the cytoskeletal protein brain spectrin.

### EXAMPLE II

### ASSAY FOR BDP IN THE BRINDLED MOUSE, AN HEREDITARY DEGENERATIVE CONDITION

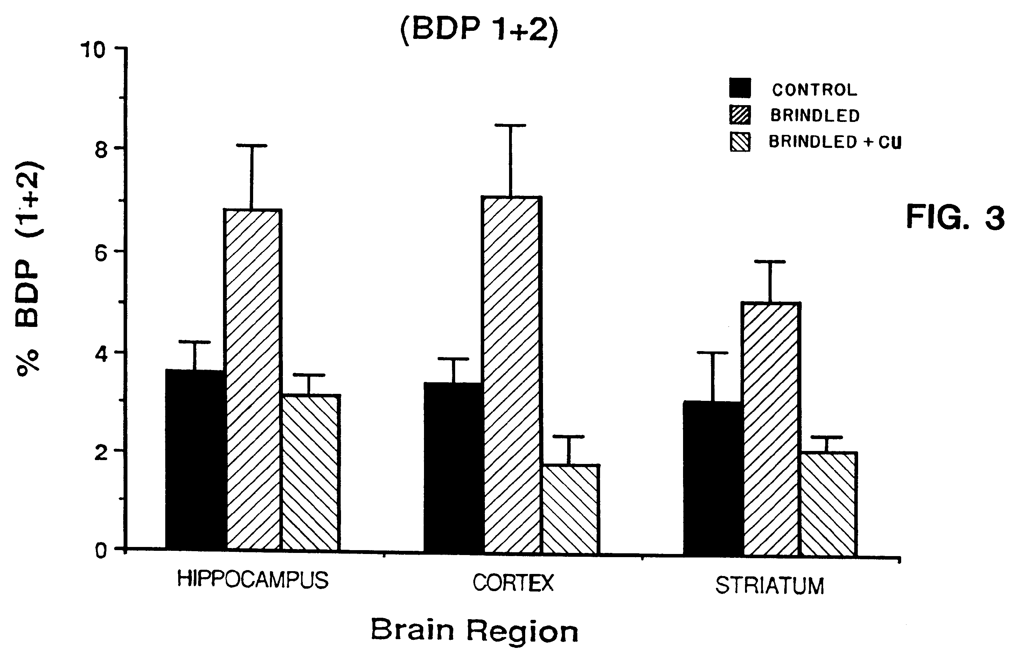

Samples from the hippocampus, cortex and striatum of 12 day old mouse pups were processed as described in Example I. The experimental groups were control mice, Brindled mice, and Brindled mice receiving supplemental copper, a treatment which prevents the premature death which otherwise occurs. Brindled mice are characterized in that they have a copper deficit which, untreated causes normal degradation. As can be seen in Figure 3 spectrin BDP's are elevated in the pathological condition and this elevation is blocked by the copper supplement.

### EXAMPLE III

### ASSAY FOR BDP AFTER EXPOSURE TO THE INDUSTRIAL TOXIN TRIMETHYLTIN (TMT)

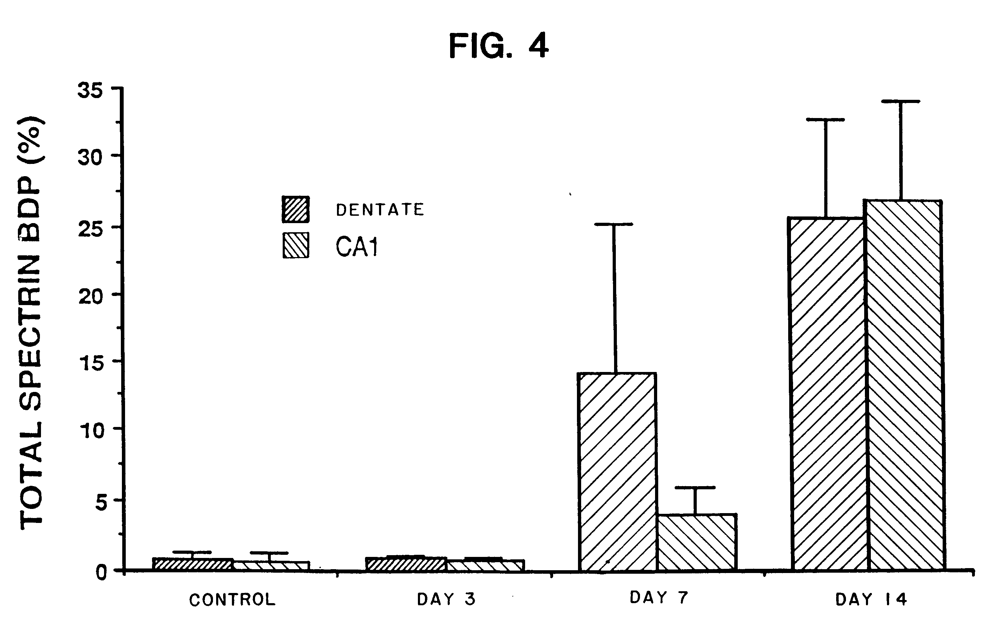

Rats were injected (i.p.) with 10 mg TMT/kg body weight. At various times post treatment the dentate gyrus and CAl regions of the hippocampus were removed and analyzed for BDP's as described in Example I. Massive increases in BDP's were noted in regions most at risk to this toxin (See Balabin et al., Neurosa 26:337-361 (1988), which is incorporated herein by reference, as depicted in Figure 4.

### EXAMPLE IV

### ASSAY FOR BDP FOLLOWING ISCHEMIA

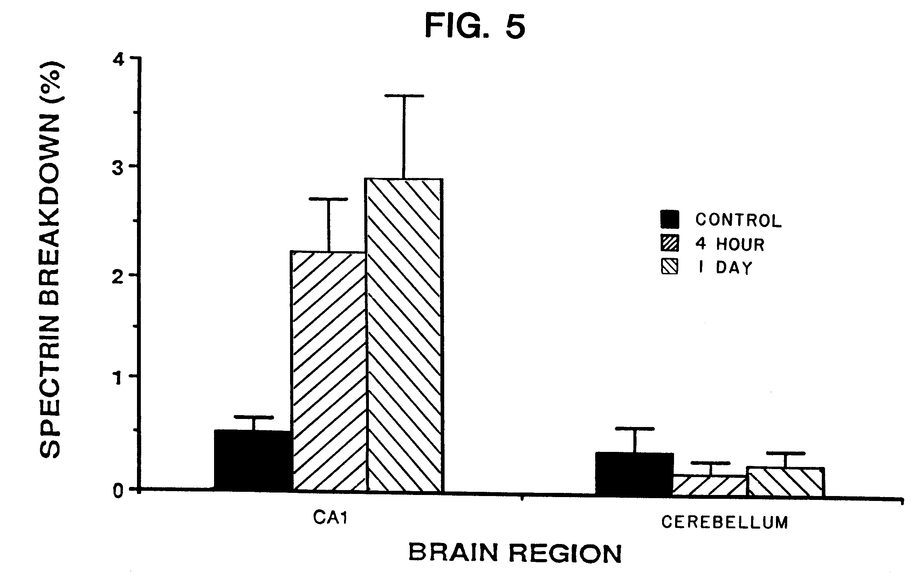

Carotid arteries were clamped for 10 minutes to interrupt the principle blood flow to the cortex in Mongolian gerbils. Samples of the CA1 hippocampal region taken either 4 or 24 hours after ischemia showed elevated BDP's compared to control animals, as shown in Figure 5. The blood supply to the cerebellum was not interrupted and this structure shows no such increase. Analysis of BDP levels was as described in Example I.

Spectrin is found in a variety of other tissues. For example, similar BDP's of spectrin have been observed in blood platelets (Fox et al., Blood 69:537-545 (1987)) and intestinal brush border cells (Glenney et al., PNAS 79:4002-4005 (1982)). The analysis described above can be extended to other tissues and fluids. The following tissue taken from rats have been examined using the methods described above, and found to exhibit spectrin and BDP's: submandibular gland, brush border, testes, thymus, skeletal muscular, heart muscle, lung, liver, spleen, adrenal gland, kidney, brain. Additionally humans, gerbils and mice have also been determined to contain spectrin and BDP's, suggesting that both are common to animals.

## Claims

1. A method of detecting cellular death or degradation in a subject comprising:
(a) analyzing a biological sample from the subject for the presence of spectrin breakdown products; and
(b) comparing the quantity of said spectrin breakdown products to the quantity of said spectrin breakdown products in a normal subject, wherein an increased level of spectrin breakdown products indicates cellular death or degradation in the subject,
characterized in that the analyzed spectrin breakdown products are two immunoreactive peptides having an apparent molecular weight of about 150,000 and 155,000 Daltons, respectively.

2. The method of claim 1, wherein the quantity of spectrin breakdown products in the normal subject is substantially undetectable.

3. The method of claim 1, wherein the biological sample comprises cellular tissue from a mammal and wherein the cellular death or degradation has occurred among the cells of the sample.

4. The method of claim 1, wherein the biological sample is selected from the group consisting of cerebrospinal fluid and a component of blood.

5. The method of claim 1, wherein the cellular death or degradation results from a non-pathological cellular insult.

6. A method of claim 5, wherein the cellular insult is selected from the group consisting of a trauma, ischemia, lesions, and exposure to toxins.

7. A method of claim 5, wherein the cellular insult is to the nervous system, and wherein the biological sample comprises neural tissue or cerebrospinal fluid.

8. The method of claim 1, wherein the cellular death or degradation results from a pathology.

9. The method of claim 8, wherein the pathology affects the nervous system.

10. The method of claim 9, wherein the pathology is selected from the group consisting of Alzheimer's disease, Parkinson's disease and muscular dystrophy, and wherein the biological sample comprises neural tissue or cerebrospinal fluid.

11. The method of claim 1, wherein the step of analyzing the biological sample for the presence of spectrin breakdown products comprises contacting a spectrin breakdown product in the sample with a detectably labeled antibody.

12. The method of claim 1, wherein the step of analyzing the biological sample comprises the steps of:
(a) exposing the sample to an electrical gradient so as to separate the components in such a way that spectrin breakdown products are separated from spectrin;
(b) contacting the separated components with a detectably labeled antibody that binds to a spectrin breakdown product; and
(c) determining the presence of antibody binding, wherein the presence of the antibody binding indicates the presence of spectrin breakdown products.

13. A method of claim 1, wherein the step of analyzing the biological sample comprises the steps of:
(a) exposing the sample to an electrical gradient so as to separate the components in such a way that spectrin breakdown products are separated from spectrin;
(b) staining a separated product with a stain which visualizes the product; and
(c) determining the presence of stain binding, wherein the presence of the stain indicates the presence of spectrin breakdown products.

14. The method of claim 1, wherein the detection occurs prior to manifestation of symptoms from the cellular death or degradation.

## Patentansprüche

1. Verfahren zum Nachweis von Zell-Tod oder Zell-Abbau in einem Subjekt, bei dem man
(a) eine biologische Probe des Subjekts auf die Anwesenheit von Spektrin-Abbauprodukten hin analysiert; und
(b) die Menge der besagten Spektrin-Abbauprodukte mit der Menge besagter Spektrin-Abbauprodukte bei einem gesunden Subjekt vergleicht, wobei ein erhöhter Spiegel von Spektrin-Abbauprodukten auf Zell-Tod oder Zell-Abbau bei der Person hindeutet,
dadurch gekennzeichnet, daß die analysierten Spektrin-Abbauprodukte zwei immunreaktive Peptide mit einem apparenten Molekulargewicht von ungefähr 150 000 bzw. 155 000 Daltons sind.

2. Verfahren gemäß Anspruch 1, bei dem die Menge der Spektrin-Abbauprodukte in der gesunden Person im wesentlichen unterhalb der Nachweisbarkeits-Grenze liegt.

3. Verfahren gemäß Anspruch 1, bei dem die biologische Probe Zellgewebe eines Säugers enthält und bei dem der Zell-Tod oder Zell-Abbau unter den Zellen der Probe stattgefunden hat.

4. Verfahren gemäß Anspruch 1, bei dem die biologische Probe aus der Gruppe bestehend aus cerebrospinaler Flüssigkeit und einer Blutkomponente ausgewählt wurde.

5. Verfahren gemäß Anspruch 1, bei dem der Zell-Tod oder Zell-Abbau aus einer nicht-pathologischen zellulären Schädigung resultiert.

6. Verfahren gemäß Anspruch 5, bei dem die zelluläre Schädigung aus der Gruppe bestehend aus Trauma, Ischämie, Läsionen, und Kontakt mit Toxinen stammt.

7. Verfahren gemäß Anspruch 5, bei dem die zelluläre Schädigung das Nervensystem betrifft, und bei dem die biologische Probe Nervengewebe oder cerebrospinale Flüssigkeit enthält.

8. Verfahren gemäß Anspruch 1, bei dem der Zell-Tod oder Zell-Abbau eine pathologische Ursache hat.

9. Verfahren gemäß Anspruch 8, bei dem die Erkrankung das Nervensystem betrifft.

10. Verfahren gemäß Anspruch 9, bei dem die Erkrankung der Gruppe bestehend aus Alzheimers-Krankheit, Parkinsons-Krankheit und Muskeldystrophie angehört und bei dem die biologische Probe Nervengewebe oder cerebrospinale Flüssigkeit enthält.

11. Verfahren gemäß Anspruch 1, bei dem die Stufe, in der die biologische Probe auf die Anwesenheit von Spektrin-Abbauprodukte hin analysiert wird, darin besteht, daß man ein Spektrin-Abbauprodukt in der Probe mit einem nachweisbar markierten Antikörper in Kontakt bringt.

12. Verfahren gemäß Anspruch 1, bei dem die Stufe, in der die biologische Probe analysiert wird, die folgenden Schritte umfaßt:
(a) Anlegen eines elektrischen Gradienten an die Probe, um die Komponenten auf solche Weise zu trennen, daß Spektrin-Abbauprodukte von Spektrin getrennt werden;
(b) Kontaktieren der getrennten Komponenten mit einem nachweisbar markierten Antikörper, welcher an Spektrin-Abbauprodukte bindet; und
(c) Bestimmen der Gegenwart von gebundenem Antikörper, wobei die Gegenwart von gebundenem Antikörper auf das Vorhandensein von Spektrin-Abbauprodukten hinweist.

13. Verfahren gemäß Anspruch 1, bei dem die Stufe, in der die biologische Probe analysiert wird, die folgenden Schritte umfasst:
(a) Anlegen eines elektrischen Gradienten an die Probe, um die Komponenten auf solche Weise zu trennen, daß Spektrin-Abbauprodukte von Spektrin getrennt werden;
(b) Kontaktieren der getrennten Komponenten mit einem nachweisbar markierten Antikörper, welcher an Spektrin-Abbauprodukte bindet; und
(c) Bestimmen der Gegenwart von gebundenem Antikörper, wobei die Gegenwart von gebundenem Antikörper auf das Vorhandensein von Spektrin-Abbauprodukten hinweist.

14. Verfahren gemäß Anspruch 1, bei dem der Nachweis vor der Manifestation von Symptomen durch Zell-Tod oder Zell-Abbau erfolgt.

## Revendications

1. Procédé de détection de la mort et de la dégradation cellulaire chez un patient comprenant :
(a) la recherche de la présence de produits de dégradation de la spectrine dans un échantillon biologique et
(b) une comparaison de la quantité de produits de dégradation de la spectrine avec la quantité de produits de dégradation de la spectrine chez un sujet normal, un taux plus élevé de produits de dégradation de la spectrine indiquant une mortalité ou une dégradation cellulaire chez le patient,
caractérisé en ce que les produits de dégradation de la spectrine analysés sont deux peptides immunoréactifs d'un poids moléculaire apparent d'environ 150.000 à 155.000 daltons, respectivement.

2. Procédé selon la revendication 1, dans lequel la quantité de produits de dégradation de la spectrine chez un sujet normal est pratiquement indécelable.

3. Procédé selon la revendication 1, dans lequel l'échantillon biologique comprend un tissu cellulaire de mammifère et dans lequel la mort et de la dégradation cellulaire est survenue au sein des celules de l'échantillon.

4. Procédé selon la revendication 1, dans lequel l'échantillon biologique est choisi au sein d'un groupe constitué par le liquide céphalorachidien et un composant du sang.

5. Procédé selon la revendication 1, dans lequel la mort ou la dégradation cellulaire résulte d'une lésion cellulaire non pathologique.

6. Procédé selon la revendication 5, dans lequel l'agent nocif pour les cellules est choisi au sein d'un groupe constitué par un traumatisme, une ischémie, des lésions et une exposition à des toxines.

7. Procédé selon la revendication 5, dans lequel l'agent nocif pour les cellules est dirigé vers le système nerveux et l'échantillon biologique comprend un tissu neural ou le liquide céphalorachidien.

8. Procédé selon la revendication 1, dans lequel la mort ou la dégradation cellulaire résulte d'une pathologie.

9. Procédé selon la revendication 8, dans lequel la pathologie affecte le système nerveux.

10. Procédé selon la revendication 9, dans lequel la pathologie est choisie parmi la maladie d'Alzheimer, la maladie de Parkinson et la dystrophie musculaire et dans lequel l'échantillon biologique comprend le tissu neural ou le lquide céphalorachidien.

11. Procédé selon la revendication 1, dans lequel l'étape d'analyse de la présence de produits de dégradation de la spectrine dans l'échantillon biologique comprend la mise en contact des produits de dégradation de la spectrine présents dans l'échantillon avec un anticorps marqué détectable.

12. Procédé selon la revendication 1, dans lequel l'étape d'analyse de l'échantillon biologique comprend les étapes consistant à :
(a) exposer l'échantillon à un champ électrique pour en séparer les composants, de manière à séparer la spectrine de ses produits de dégradation;
(b) mettre en contact les constituants isolés avec un anticorps marqué détectable qui se lie à un produit de dégradation de la spectrine; et
(c) déterminer la liaison à l'anticorps, celle-ci indiquant la présence de produits de dégradation de la spectrine.

13. Procédé selon la revendication 1, dans lequel l'étape d'analyse de l'échantillon biologique comprend les étapes consistant à :
(a) exposer l'échantillon à un champ électrique pour en séparer les composants, de manière à séparer la spectrine de ses produits de dégradation;
(b) colorer un produit isolé avec un colorant permettant de le visualiser; et
(c) déterminer la liaison au colorant, celle-ci indiquant la présence de produits de dégradation de la spectrine.

14. Procédé selon la revendication 1, dans lequel la détection est réalisée avant l'apparition de symptômes de mort ou de dégradation cellulaire.
